# EUROPEAN PATENT APPLICATION

(11) **EP 1 872 814 A1**
(43) Date of publication of application: **02.01.2008**
(21) Application number: 06116174.1
(22) Date of filing: 27.06.2006
(51) Int. Cl.: A61M 1/28, A61M 1/34, A61M 1/16, G06F 19/00, B65D 77/06

(54) **Medical system for performing a fluidic treatment**

(71) Applicant: DEBIOTECH S.A., 1004 Lausanne (CH)
(72) Inventor: Evering, Hans-Gerd, 1802, Corseaux (CH); Neftel, Frédéric, Dr., 1005, Lausanne (CH)
(74) Representative: Grosfillier, Philippe

(57) **Abstract**

The invention relates to a medical system for the delivery and/or the extraction of fluid to and/or from a patient. The medical device comprises one user interface unit (1), one mechatronic interface unit (2) including pumping means and at least one patient line (7) with one end connected to said mechatronic interface unit (2) and the other end adapted to be connected to a patient; said medical device being characterized by the fact that said user interface unit (1) is forming a separate unit, distinct from the remaining elements of said medical device, said user interface unit (1) being adapted to be used in a remote location with respect to said mechatronic interface unit (2), said user interface unit (1) and said mechatronic interface unit communicating each others with a cable or a wireless connection.

The system according to the invention may advantageously be used in fluid therapies such as Peritoneal Dialysis (PD), such CAPD, APD, CFPD and combinations and Haemodialysis (HD), such as HD, HF, HDF, HHD and combinations.

## Description

### Field of invention

The present invention generally relates to medical systems for performing a fluidic treatment. More specifically, the present invention relates to the delivery and/or extraction of fluid to and/or from a patient, e.g. for performing dialysis therapies.

### State of the art

Haemodialysis and Peritoneal Dialysis are forms of medical applications to treat patients suffering from kidney diseases. More and more of these treatments are performed at patients home environments, especially PD treatments. It is also foreseeable, that with the availability of improved HD device in the sense of safety ness and ease of use the number of Home Haemodialysis (HHD) devices will increase over time.

The current, available devices for such treatments are still dominated in their design for the use in hospitals and the hospital environmental needs. They are made of several functional units (pumping unit, user interface units, container unit, etc...) which are all integrated in one single enclosure. In addition, the state of the art devices are more or less large and heavy as a result of integration of all required functions into one device. From the outer appearances, all devices are clear identification able as medical devices. Apart from the endeavour to make such devices smaller and transportable for the patients home environments, new technical solutions are not presented and new technologies did not find entrance into this field of application to improve patients ease of use, patients comfort and home environmental needs.

There is therefore a need to improve the state of the art devices.

### Description of the Invention

This need is met with the present invention which relates to a medical system for the delivery and/or the extraction of fluid to and/or from a patient, said medical device comprising one user interface unit, one mechatronic interface unit (i.e. a unit combining mechanical, electronic and software elements) including pumping means and at least one patient line with one end connected to said mechatronic interface unit and the other end adapted to be connected to a patient; said medical device being characterized by the fact that said user interface unit is forming a separate unit, distinct from the remaining elements of said medical device, said user interface unit being adapted to be used in a remote location with respect to said mechatronic interface unit, said user interface unit and said mechatronic interface unit communicating each others with a cable or a wireless connection.

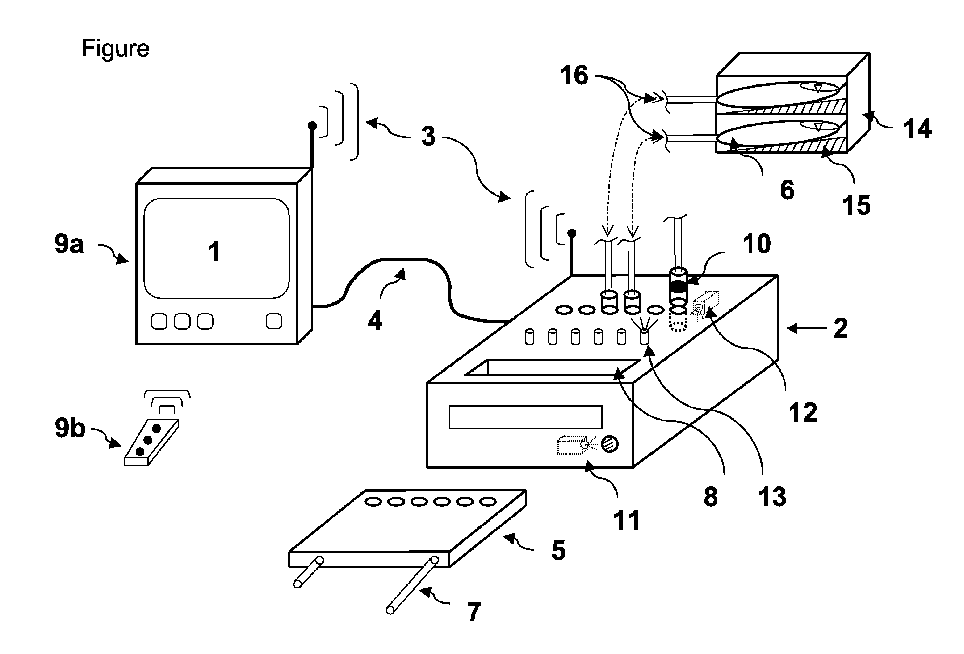

An embodiment of the invention, using a peritoneal dialysis system (PD) is discussed below and illustrated by the figure :
Such a system combines a disposable **5** and PD solutions bags **6** with a electromechanical component - entitled as mechatronic interface **2 -** and a user interface unit **1,** all those elements being functionally connected. The disposable **5** is inserted into the mechatronic interface **2** at the beginning of the treatment. During the treatment, no "physical" contribution from the patient is requested, except the possible activation of the user interface unit **1.**
As it can be seen on the figure, the user interface unit 1 is a separate element, distinct and distant from the remaining elements of the medical device (mechatronic interface, containers, disposable, etc...). It becomes therefore possible to place all those remaining elements in a suitable area which is distant from the patient. This configuration offers several advantages over the state of the art device, in particular :
   - Medical device may be stored close to solution bags and drainage facilities,
   - Noise reduction thanks to the distance between treatment area and operating area (e.g. the noisy pumping unit is placed away from the patient),
   - Hiding reasons to be more comfortable during daily treatments with visitors and nocturnal treatments with partners.

The same principle in dividing functional units is applicable to HD or other similar fluidic medical systems where the fluid preparation compartment as a standalone part of the device can be separated, even in a separate room from the treatment area. The required extracorporeal blood circuit (EBC) should be preferably part of the user interface, with a potential outcome to reduce size and increase safety ness.

To program the treatment before its start or adapt or act on it when in use, the user operates user interface unit **1** which, as seen before, is the only element of the medical system which may stay close to the patient during the treatment.

Therefore, a first advantage of this invention results from this separate user interface unit **1** which consists mainly out of silent electronic and software components. The electrical communication between the user interface **1** and mechatronic interface **2** is performed with electrical wires **4** and / or preferably with wireless communication technology **3.** For the hydraulic communication (in the field of PD application for example), a extended patient line **7** is provided in order to "connect" the patient with the mechatronic interface **2.**

To address the clinical needs of such a system, the user interface unit **1** may be fixed to the mechatronic interface **2,** e.g. for storage or transportation or use in clinical environments. Another advantage of the invention can be seen in training purposes, where the trainer and the patient can share the user interface unit **1** without interfering with fluid lines, solution bags and drainage systems.

### User Interface Appearance

The described user interface unit **1** can be designed with the following technical features. The appearance of this user interface is different from existing medical devices and is closer to a device design, which may already exists in patients home. Advantageously, this design solutions are chosen from the appearances of flat screen TV, radio clock, palm top and / or note pads as examples. Also, beneficial will be the appearance of this user interface unit **1,** which will be more suitable for the patient home environment. Finally, the separation from user interface unit **1** and mechatronic interface unit **2** of a medical device offers more flexibility for design solutions.

### Ease of Use

Additionally, with the benefit that the patient is already familiar with this technologies and he knows how to operate it, confidence will increase and human factor objectives will improve.

### Differentiation between initial setup (preparation) and use during treatment

To operate the user interface unit **1,** technologies like Touch Screens, I-Drives, Remote Controls and even Voice operating system can be chosen. Another fundamental advantage in this invention is the differentiation between initial treatment setup operations via user interface unit **1** such as touch, buttons, etc **9a** and patient comfort operations such as "Stop treatment for short time to relief pain situations", "Change parameters", etc., especially during nocturnal treatments utilizing a additional function in having a remote control system, voice operating system or others **9b** to avoid switching on lights and partner disturbance.

### Wireless Communication and Power Supply

To benefit from all possibilities of this invention, the communication between the user interface unit **1** and the mechatronic interface unit **2** is preferably equipped with wireless communication technology **3** and additional wireless electrical power supply for the user interface unit **1.** Alternatively, electrical hardware wiring **4** can also offer most of all mentioned advantages.

The present invention includes furthermore technical solutions with advantages for patient comfort and ease of use.

E.g. for PD treatments, it is state of the art to connect PD solution bags **6** to a disposable **5** with solution bag lines, equipped with connectors. To perform different treatment modes, it is required that these medical devices identify the type of solution bags in size and content and the connected fluid port on the disposable **5.** This invention presents a system which, for example, is using colour coded connectors **10** for solution bags which are readable and recognizable by LED technology. Alternatively to the colour coding may be replaced by a integrated RFID tag to identify the PD solution bag types.

### Insertion Guidance

In the absence of a user interface unit **1** due to the moveable functionality, it is required to support the solution bag connection. Therefore, the system according to the invention is equipped with a electronic reading system as a part of the mechatronic interface **2.** This electronic reading system can be a single reader **11** preferably located close to the handling area, or a multi functional reader for each individual connecting port, entitled as port reader **12.** These readers should be suitable to identify colour coded and/or RFID coded and/or bar code coded connectors **10.**

In identifying the type of bag by placing the bag connector **10** into the single reader **11** or one of the port readers **12,** a LED light **13** indicates the user in the absence of the user interface unit **1,** in which port this connector needs to be placed for connection in conjunction with the programmed treatment parameters. The specific port readers **12** itself can be used additional to verify the correct bag type within the correct fluid port position. This bag recognition system avoids any trial and error issues and supports the ease of use.
A small explanation display can also be part of the mechatronic interface 2, although it does not need to be as completed as for the user interface 1. Alternatively, the user interface can be brought in close contact to the mechatronic interface 2 during tube connection and moved close to the patient during use.

### Medical Fluid Solution Bags - Handling and Management

The separation of the user interface unit **1** from the mechatronic interface **2** offers also several new advantages for the medical fluid solution bag handling and management. Firstly, for example for PD treatments, the PD solution bags **6,** the drainage system, either with bags, container or tube drainage can be handled and managed in more suitable ways, depending on patients home environments. Operating the mechatronic interface **2** in the same area of bag storage and drainage facilities, less bag transportation from and back to the treatment area is required. A second advantage could be made by the delivery of PD solution bags in cartons or container **14,** where one or more bags are stored on a slope **15** under a specific angle in relation to the tube outlet of such bags. These solution bags should be equipped with a fixed tube line **16** and a connector **10,** which is preferably access able from the carton or container **13** without moving the PD solution bags **6.** Such system is beneficial for handling (may include a built-in handle as well) and connection. Furthermore, systematically included air in PD solution bags will be not be sucked out of these bags during treatment and will remain in the PD solution during emptying.

A third advantage of this invention can be seen in less interference of the treatment area for nocturnal treatments, which are mostly bed rooms in not rinsing fluidics, which may drop on carpet floors. Such system creates more space at the treatment area and the overall appearance may look different from hospital environments.

In addition, the mechatronic interface unit may be close to an evacuation for the fluid to be drained, so that the patient does not need to transport such heavy drainage container which is commonly used.

### Noise Reduction for Patient Home Environments

Another, fundamental advantage offers this invention in accordance to noise disturbance, especially during night time treatments. By ensuring, that all electromechanical elements, which may create noise, are implemented in the mechatronic interface **2** a noise reduction can be made simply by distance to this source of noise.

### Mechatronic Interface Design, Technical upgrades, Costs

Another, fundamental advantage of this invention relates to the different design requirements for user interface unit **1** and mechatronic interface unit **2.** Especially, the mechatronic interface unit **2** can be designed to address human factors requirements only for functionalities. In fact, the enclosures can be simple and functional, any upgrade of technical functionalities can be performed without interfering with the user interface system.

Of course the invention is not limited to the above discussed examples.

## Claims

1. Medical system for the delivery and/or the extraction of fluid to and/or from a patient, said medical device comprising one user interface unit (1), one mechatronic interface unit (2) including pumping means and at least one patient line (7) with one end connected to said mechatronic interface unit (2) and the other end adapted to be connected to a patient; said medical device being **characterized by** the fact that said user interface unit (1) is forming a separate unit, distinct from the remaining elements of said medical device, said user interface unit (1) being adapted to be used in a remote location with respect to said mechatronic interface unit (2), said user interface unit (1) and said mechatronic interface unit communicating each others with a cable or a wireless connection.

2. Medical system according to claim 1 wherein said mechatronic interface unit (2) is including a fluid distribution system, mixing means for mixing the content of at least one fluid container (6) and/or fluid heating system to warm the fluid.

3. Medical system according to claim 1 or 2 wherein said patient line (7) is made of at least one single lumen tube and/or one multiple lumen tube.

4. Medical system according to anyone of the previous claims furthermore comprising a fluid container (14) communicating with, and situated close to, said mechatronic interface unit (2).

5. Medical system according to anyone of the previous claims furthermore comprising a user remote control system (9b) which is adapted to communicate with said user interface unit (1).

6. Medical system according to anyone of the previous claims wherein said mechatronic interface unit (2) comprises fixing means which are adapted to fix said user interface unit (1) when the complete medical system is moved and/or utilized in hospitals.

7. Medical system according to anyone of the previous claims wherein said mechatronic interface unit (2) comprises or is connected to at least one balance to measure the volume of fluid pumped into or from a patient.

8. Medical system according to claim 7 wherein the connection to the balance is wireless.

9. Medical system according to anyone of the previous claims comprising several fluid lines (16) with respective connectors (10) fixed at one of their ends, said mechatronic interface (2) containing several ports which are adapted to receive said connectors (10); said medical system furthermore comprising an identification system which is adapted to guide and identify the port on which a specific fluid line (16) has to be connected.

10. Medical system according to claim 9 wherein said identification system comprises identification elements in said connectors (10) and visual indicators situated close to said ports, each visual indicator being adapted to show different colors in order to guide and to qualify the type of connection made or required.

11. Medical system according to claim 9 or 10 wherein said identification system comprises identification elements in said connectors (10) and at least one audio indicator adapted to produce different sounds in order to guide and to qualify the type of connection made or required.

12. Medical system according to claim 10 or 11 wherein the identification element is an electronic device such as a RFID tag or a bar code or a color code.

13. Medical system according to anyone of previous claims 9 to 11 furthermore comprising a detection device able to read the identification element prior to the connection in order to activate the visual and/or audio indicator of the appropriate port.

14. Medical system according to anyone of the previous claims 2 to 12 wherein said fluid container (6) is in a housing (14), said fluid container (6) is placed at an angle (15) with respect to the horizontal when the housing (14) is placed on an horizontal surface.

15. Medical system according to claim 14 wherein said angle is of at least 10°.

16. Medical system according to claim 13 or 14 wherein said fluid container (6) is made out of flexible polymer.

17. Medical system according to anyone of claim 14 to 16 wherein said housing is made out of cardboard.

18. Medical system according to anyone of claim 14 to 16 wherein the fluid line (16) is fixed to said fluid container (6) in such a way that it may be removed from the system without having to access or remove said fluid container (6) from said housing (14).
